# EUROPÄISCHE PATENTSCHRIFT

(11) **EP 2 258 290 B1**
(45) Veröffentlichungstag und Bekanntmachung des Hinweises auf die Patenterteilung: **31.07.2013**
(21) Anmeldenummer: 10001409.1
(22) Anmeldetag: 11.02.2010
(51) Int. Cl.: A61B 17/72, A61B 17/17

(54) **Humerusnagel zur Versorgung von Frakturen des Humerus**
Humeral nail for humerus fractures
Clou huméral destiné au traitement de fractures de l'humérus

(30) Priorität: 25.02.2009 DE 102009010328
(43) Veröffentlichungstag der Anmeldung: 08.12.2010
(73) Patentinhaber: Dieter Marquardt Medizintechnik GmbH, 78549 Spaichingen (DE)
(72) Erfinder: Marquardt, Dieter, 78549 Spaichingen (DE); Mayr, Edgar, Prof. Dr. Dr. h. c., 86444 Affing (DE)
(74) Vertreter: Geitz Truckenmüller Lucht

(56) Entgegenhaltungen:
- EP-A1- 1 354 562
- EP-A1- 1 779 795
- EP-A2- 1 175 872
- WO-A1-2004/043277
- WO-A1-2005/092219
- WO-A2-2007/109302
- DE-U1- 29 907 161
- ES-A1- 2 251 888
- FR-A1- 2 781 360

## Beschreibung

Die Erfindung geht aus von einem Humerusnagel zur Versorgung von Frakturen des Humerus.

Bei Frakturen des als Humerus bezeichneten Oberamknochens ist zumeist der Humeruskopf betroffen. Zur Versorgung derartiger Frakturen ist ein Marknagel vorgesehen, der aufgrund seines Anwendungsbereiches als Humerusnagel oder Humerusmarknagel bezeichnet wird. Humerusnägel werden am proximalen Ende des Humerus in den Röhrenknochen eingetrieben und durch mehrere Fixierungselemente im proximalen und/ oder distalen Bereich mit dem Knochen verbunden. Hierzu ist der Humerusnagel im proximalen und distalen Bereich mit Bohrungen ausgestattet. Diese können quer zur Längsachse des Nagelschaftes unter einem Winkel von 90° oder von weniger als 90° verlaufen. Die Bohrungen schneiden die Längsachse des Nagelschaftes an unterschiedlichen Positionen, da sie bezüglich der Längsachse versetzt angeordnet sind. Die Bohrachse oder Längsachse der Bohrungen verläuft unter demselben Winkel oder unter verschiedenen Winkeln zur Längsachse des Nagelschaftes des Humerusnagels. Ferner können die Bohrachsen je zweier Bohrungen parallel zueinander oder unter einem von 0° verschiedenen Winkel zueinander verlaufen. Als Fixierungselemente werden beispielsweise Knochenschrauben oder Knochenstifte verwendet. Die Fixierungselemente sollen dabei eine axiale Verschiebung des Humerusnagels verhindern und den Humerusnagel gegen Torsion sichern. Ein derartiger Humerusnagel ist beispielsweise aus der EP 1 402 831 A2 bekannt. Ein ähnlicher Nagel zum Fixieren der Femurs ist aus ES 2251888 bekannt.

Als nachteilig erweist sich bei den bekannten Humerusnägeln, dass bei einer frühen Mobilisierung des Patienten der mit einem Humerusnagel versorgte Humerus insbesondere im Bereich des Humeruskopfes und der Rotatorenmanschette den auftretenden Kräften nicht standhalten kann.

Es ist daher Aufgabe der vorliegenden Erfindung, einen Humerusnagel zur Verfügung zu stellen, der eine frühe Mobilisierung des Patienten ermöglicht, indem die auf den Humerus einwirkenden Kräfte insbesondere im Bereich des Humeruskopfes und der Rotatorenmanschette abgefangen werden.

Diese Aufgabe wird durch einen erfindungsgemäßen Humerusnagel mit den Merkmalen des Anspruchs 1 gelöst. Der Humerusnagel zeichnet sich dadurch aus, dass zusätzlich zu den Bohrungen für Fixierungselemente eine Durchgangsöffnung im Nagelschaft vorgesehen ist. Diese Durchgangsöffnung dient zur Aufnahme eines zusätzlichen Stabilisierungselements. Dieses zusätzliche Stabilisierungselement ist von den stiftförmigen Fixierungselementen verschieden. Seine Länge ist typischerweise größer als die Länge der Fixierungselemente. Ein Ende des Stabilisierungselements weist bevorzugt mindestens eine Aufnahme für einen Abschnitt eines stiftförmigen Fixierungselements auf. Das Stabilisierungselement wird so weit durch die Durchgangsöffnung im Nagelschaft hindurch geschoben, dass ein in eine Bohrung des Nagelschaftes aufgenommenes Fixierungselement mit seinem einen Ende in der Aufnahme des Stabilisierungselements angeordnet ist. Damit bildet das durch die Durchgangsöffnung geführte Stabilisierungselement zusammen mit dem Fixierungselement eine zusätzliche Verstrebung des Humerusnagels.

Stabilisierungselement, Fixierungselement und Nagelschaft bilden ein Dreieck. Dabei kann die durch das Stabilisierungselement gebildete Seite auch gekrümmt sein. Durch die Verbindung zwischen dem Stabilisierungselement und dem Fixierungselement wird ein Verschieben des Stabilisierungselements in der Durchgangsöffnung verhindert. Das Stabilisierungselement sorgt dafür, dass die auf den Humeruskopf einwirkenden Kräfte abgefangen werden und die sekundäre Abkippung des Humeruskopfes vermieden wird. Dadurch wird eine zusätzliche Stabilisierung des Humerus erreicht. Eine frühe Mobilisierung des Patienten ist damit möglich. Das Stabilisierungselement weist einen gekrümmten Verlauf auf.

Der Winkel, unter dem die Durchgangsöffnung bezüglich der Längsachse des Nagelschaftes verläuft, ist bevorzugt kleiner als 90°. Bei einem geradlinigen Verlauf der Durchgangsöffnung wird der Winkel zwischen einer durch den Verlauf vorgegebenen Geraden und der Längsachse bestimmt. Bei einem gekrümmten Verlauf der Durchgangsöffnung wird zur Bestimmung des Winkels an eine durch den Verlauf vorgegebene und die Längsachse schneidende Kurve eine Tangente angelegt. Die Tangente wird an die Kurve im Schnittpunkt mit der Längsachse angelegt. Anschließend wird der Winkel zwischen der Tangente und der Längsachse bestimmt.

Der Winkel, unter dem die Bohrung verläuft, welche mit einem in dem Stabilisierungselement aufgenommenen Fixierungselement ausgestattet ist, kann unter einem Winkel von 90° oder von weniger als 90° gegen die Längsachse des Nagelschaftes verlaufen.

Bei der Aufnahme für ein Fixierungselement in dem Stabilisierungselement kann es sich beispielsweise um eine durchgängige oder nicht durchgängige Bohrung, eine Öffnung mit eckigem oder ovalem Querschnitt, eine Kerbe oder eine konische Vertiefung handeln.

Die Bohrungen im Nagelschaft können frei von einem Gewinde sein oder mit einem Gewinde ausgestattet sein. Sofern sie mit einem Gewinde ausgestattet sind, ist dieses auf ein Gewinde der Fixierungselemente abgestimmt. In diesem Fall handelt es sich bei den Fixierungselementen um Knochenschrauben.

Nach einer vorteilhaften Ausgestaltung der Erfindung verläuft die Durchgangsöffnung für das Stabilisierungselement zwischen den im proximalen Bereich vorgesehenen Bohrungen und dem distalen Ende des Nagelschaftes. Aufgrund einer Neigung der Durchgangsöffnung zur Längsachse des Nagelschaftes erstreckt sich ein durch die Durchgangsöffnung hindurch geführtes Stabilisierungselement mit seinem einen Ende bis in den Bereich der durch die Bohrungen im proximalen Bereich des Nagelschaftes eingesetzten Fixierungselemente. Das in die Durchgangsöffnung aufgenommene Stabilisierungselement bildet damit zusammen mit einem Fixierungselement eine Verbindung zwischen einem Abschnitt des Humerusnagels, der sich zwischen den proximalen Bohrungen und dem distalen Ende befindet, und dem proximalen Bereich des Humerusnagels.

Darüber hinaus kann die Durchgangsöffnung auch zwischen dem proximalen Ende des Nagelschaftes und den proximalen Bohrungen positioniert sein. Ferner besteht die Möglichkeit, die Durchgangsöffnung zwischen zwei der proximalen Bohrungen vorzusehen. Die Neigung der Durchgangsöffnung gegen die Längsachse des Nagelschaftes hängt dabei von der Position mindestens einer der Querborungen ab, wobei das in die Bohrung eingesetzte Fixierungselement mit dem Stabilisierungselement und dem Nagelschaft ein Dreieck bildet. Je kleiner der Abstand zwischen der Durchgangsöffnung und dieser Bohrung ist, um so größer ist der Winkel zwischen der Durchgangsöffnung und der Längsachse des Nagelschaftes und um so kleiner ist die durch einen Abschnitt des Nagelschaftes gebildete Seite des Dreiecks. Dabei wird davon ausgegangen, dass die Fixierungselemente eine vorgegebene Länge aufweisen und sich Fixierungselement und Stabilisierungselement an einer vorgegebenen Position kreuzen.

Nach einer weiteren vorteilhaften Ausgestaltung der Erfindung weist die Durchgangsöffnung einen gekrümmten Verlauf auf. In eine derartige Durchgangsöffnung wird ein Stabilisierungselement eingesetzt, das ebenfalls gekrümmt ist, wobei der Verlauf der Krümmung qualitativ mit der Krümmung der Durchgangsöffnung übereinstimmt. Eine durch die Durchgangsöffnung in Längsrichtung zentrisch verlaufende Kurve stimmt darüber hinaus bevorzugt hinsichtlich ihres Verlaufs quantitativ mit einer entsprechenden Kurve durch das Stabilisierungselement überein. Durch die Krümmung wird erreicht, dass das Stabilisierungselement mit seinem einen Endbereich, welcher mit einer Aufnahme für ein Fixierungselement ausgestattet ist, einen kleineren Abstand zu dem Nagelschaft aufweist, als ein Stabilisierungselement mit geradlinigem Verlauf. Dadurch wird eine Stabilisierung des Humerus auf kleinerem Raum erzielt, als bei einem Stabilisierungselement mit geradlinigem Verlauf. Darüber hinaus können bei einer Durchgangsöffnung und einem Stabilisierungselement mit gekrümmtem Verlauf die Fixierungselemente eine geringere Länge aufweisen, da der Kreuzungspunkt von Stabilisierungselement und Fixierungselement näher am Nagelschaft liegt als bei einem geradlinig verlaufenden Stabilisierungselement. Darüber hinaus kann die Durchgangsöffnung auch einen geradlinigen Verlauf aufweisen. Vorteilhafterweise stimmt der Verlauf der Durchgangsöffnung qualitativ mit dem Verlauf des Stabilisierungselements überein. Dadurch wird das Stabilisierungselement durch die Durchgangsöffnung über die gesamte Länge der Durchgangsöffnung geführt und gestützt. Ist dagegen der Verlauf der Durchgangsöffnung geradlinig und der Verlauf des Stabilisierungselements gekrümmt, so findet nur eine Unterstützung an drei Punkten statt.

Nach einer weiteren vorteilhaften Ausgestaltung der Erfindung weist die Durchgangsöffnung über ihre gesamte Länge einen konstanten Querschnitt auf.

Entsprechendes gilt für das Stabilisierungselement, insbesondere für den Abschnitt, der durch die Durchgangsöffnung hindurch geführt wird.

Nach einer weiteren vorteilhaften Ausgestaltung der Erfindung weist die Durchgangsöffnung einen länglichen Querschnitt auf. Entsprechendes gilt bevorzugt für das Stabilisierungselement. Darüber hinaus weisen sowohl die Durchgangsöffnung als auch das Stabilisierungselement in ihren Querschnitten keine Ecken auf. Dies führt zu einer kantenfreien Außenkontur des Stabilisierungselements. Der längliche Querschnitt des Stabilisierungselementes sorgt zum einen für eine hohe Stabilität und bietet zum anderen ausreichend Platz beziehungsweise Raum für eine Aufnahme für eines oder mehrere Fixierungselemente.

Nach einer weiteren vorteilhaften Ausgestaltung der Erfindung schneiden sich die Längsachse mindestens einer der Bohrungen einerseits und die Kurve, welche durch den Verlauf der Durchgangsöffnung vorgegeben ist, andererseits. Dies gilt sowohl für eine geradlinige Durchgangsöffnung als auch für eine Durchgangsöffnung mit gekrümmtem Verlauf. Der Schnittpunkt der Längsachse der Bohrung mit der durch die Durchgangsöffnung vorgegebene Kurve sorgt dafür, dass ein in die Durchgangsöffnung eingesetztes Stabilisierungselement und ein in eine Bohrung eingesetztes Fixierungselement einander berühren oder kreuzen.

Nach einer weiteren vorteilhaften Ausgestaltung der Erfindung beträgt der Winkel der Durchgangsöffnung gegen die Längsachse des Nagelschaftes in bevorzugter Weise zwischen 10° und 60°. Bei einem gekrümmten Verlauf der Durchgangsöffnung entspricht dies dem Winkel zwischen einer im mittleren Abschnitt der Durchgangsöffnung an die Kontur angelegten Tangente und der Längsachse des Nagelschaftes.

Nach einer weiteren vorteilhaften Ausgestaltung der Erfindung handelt es sich bei der Aufnahme in dem Stabilisierungselement um eine Bohrung mit oder ohne Gewinde. Im Falle einer Gewindebohrung ist das Gewinde auf ein Gewinde der Fixierungselemente abgestimmt. Eine als Gewindebohrung ausgebildete Aufnahme hat den Vorteil, dass das aufgenommene Fixierungselement auch in Richtung seiner Längsachse fixiert ist und nicht nur seitlich zur Längsachse.

Nach einer weiteren vorteilhaften Ausgestaltung der Erfindung befindet sich die Aufnahme an einem Ende des Stabilisierungselementes. Es handelt sich bevorzugt um eine durchgängige Bohrung, insbesondere um eine Gewindebohrung, oder um eine Öffnung. Sie erstreckt sich damit durch das Stabilisierungselement hindurch und kann somit einen möglichst großen Abschnitt eines Fixierungselements aufnehmen.

Nach einer weiteren vorteilhaften Ausgestaltung der Erfindung weist das Stabilisierungselement an dem diesem Ende abgewandten Endbereich einen Abschnitt auf, dessen Querschnitt größer ist, als der Querschnitt der Durchgangsöffnung. Dieser Abschnitt dient als Anschlag beim Einführen des Stabilisierungselements in die Durchgangsöffnung. Ist das Stabilisierungselement soweit durch die Durchgangsöffnung hindurch geschoben, dass der Abschnitt mit dem größerem Querschnitt am Nagelschaft anliegt, so ist ein weiteres Einschieben des Stabilisierungslementes verhindert. Dem Arzt wird beim Einsetzen dadurch angezeigt, dass das Stabiliserungselement bis zu der vorgesehenen Position vorgedrungen ist. In dieser Position kann die Aufnahme in dem Stabilisierungselement ein in eine Bohrung des Nagelschaftes eingesetztes Fixierungselement aufnehmen.

Nach einer weiteren vorteilhaften Ausgestaltung der Erfindung ist das Stabilisierungselement mit einer keilförmigen Schneide ausgestattet. Die Schneide kann geschärft sein. Die keilförmige Schneide ist bevorzugt an einer Stirnseite des Stabilisierungselements angeordnet. Sie erleichtert das Eintreiben des Stabilisierungselements in den Knochen.

Weitere Vorteile und vorteilhafte Ausgestaltungen der Erfindung sind der nachfolgenden Beschreibung, der Zeichnung und den Ansprüchen zu entnehmen.

### Zeichnung

In der Zeichnung sind zwei Ausführungsbeispiele eines erfindungsgemäßen Humerusnagels dargestellt. Es zeigen:
- Figur 1: erstes Ausführungsbeispiel eines Humerusnagels mit kurzem Stabilisierungselement in einer Seitenansicht,
- Figur 2: Humerusnagel gemäß Figur 1 in perspektivischer Darstellung,
- Figur 3: Humerusnagel gemäß Figur 1 mit zwei Knochenschrauben in Seitenansicht,
- Figur 4: Humerusnagel gemäß Figur 3 im Längsschnitt,
- Figur 5: Humerusnagel gemäß Figur 3 in einer um 90° gedrehten Seitenansicht,
- Figur 6: Humerusnagel gemäß Figur 1 mit Humerus,
- Figur 7: Nagelschaft des Humerusnagels gemäß Figur 1 in zwei Seitenansichten und einem Längsschnitt,
- Figur 8: Stabilisierungselement des Humerusnagels gemäß Figur 1 in verschiedenen Ansichten und in Schnittdarstellung,
- Figur 9: zweites Ausführungsbeispiel eines Humerusnagels mit langem Stabilisierungselement in einer Seitenansicht,
- Figur 10: Humerusnagel gemäß Figur 9 in perspektivischer Darstellung,
- Figur 11: Humerusnagel gemäß Figur 9 mit zwei Knochenschrauben in Seitenansicht,
- Figur 12: Humerusnagel gemäß Figur 11 im Längsschnitt,
- Figur 13: Humerusnagel gemäß Figur 11 in einer um 90° gedrehten Seitenansicht,
- Figur 14: Humerusnagel gemäß Figur 9 mit Humerus,
- Figur 15: Stabilisierungselement des Humerusnagels gemäß Figur 9 in verschiedenen Ansichten und in Schnittdarstellung,
- Figur 16: Zielgerät mit Bohrer in Seitenansicht,
- Figur 17: Zielgerät mit Schutzhülse in perspektivischer Ansicht.

### Beschreibung der Ausführungsbeispiele

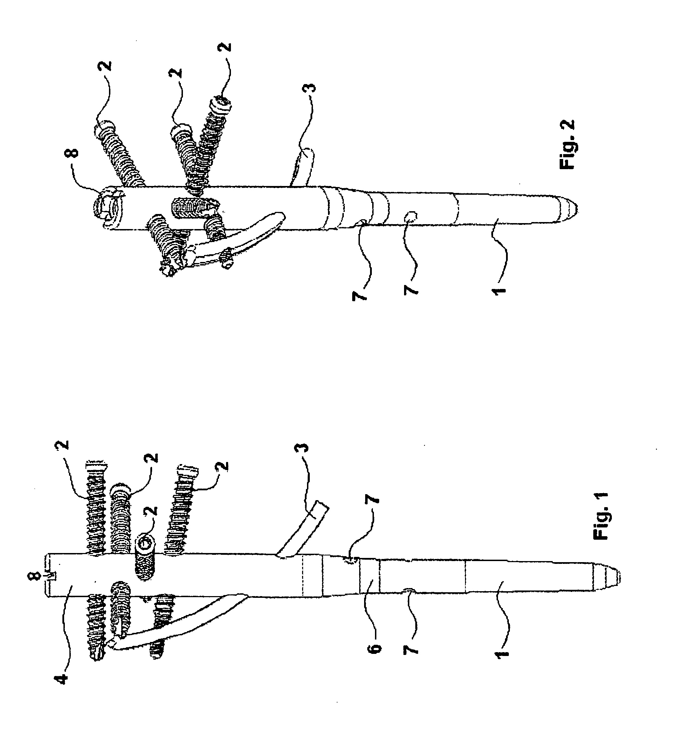

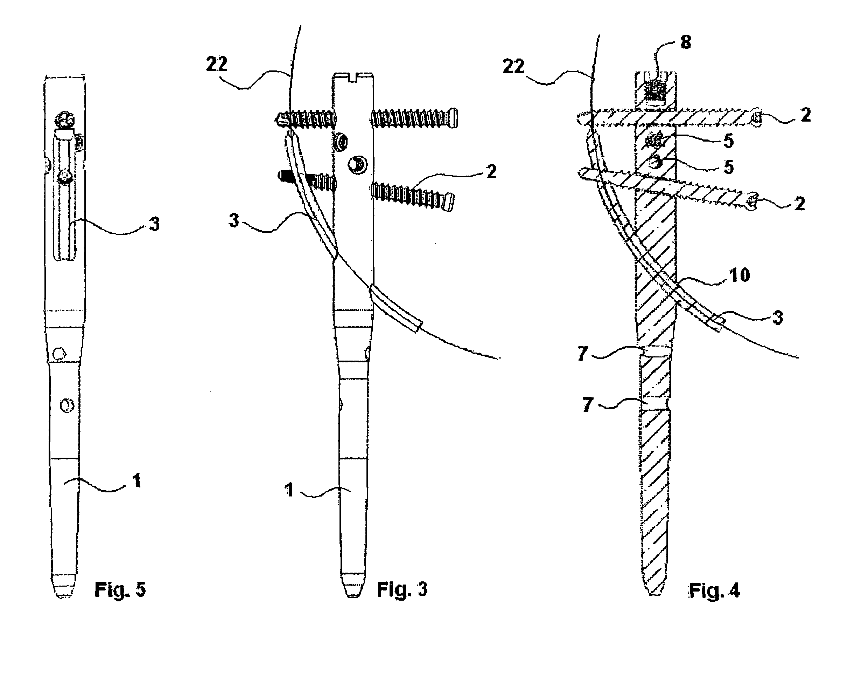

In den Figuren 1 bis 8 ist ein erstes Ausführungsbeispiel eines Humerusnagels mit einem Nagelschaft 1, vier als Fixierungselemente 2 dienende Knochenschrauben 2 und einem Stabilisierungselement 3 dargestellt. Der Nagelschaft ist in seinem proximalen Bereich 4 mit insgesamt vier Bohrungen 5 für Fixierungselemente 2 ausgestattet. Die Bohrungen 5 im proximalen Bereich des Nagelschaftes 1 werden auch als proximale Bohrungen 5 bezeichnet. In den Figuren 1, 2 und 6 sind in allen vier Bohrungen 5 Fixierungselemente 2 angeordnet. In den Figuren 3, 4 und 5 sind nur zwei der Bohrungen 5 mit Fixierungselemente 2 ausgestattet. In dem distalen Bereich 6 des Nagelschaftes 1 befinden sich zwei weitere Bohrungen 7 für Fixierungselemente. Diese Bohrungen werden als distale Bohrungen 7 bezeichnet. In der Zeichnung sind die Bohrungen 7 nicht mit Fixierungselemente versehen. Die Bohrungen 5 und 7 befinden sich an unterschiedlichen Positionen des Nagelschaftes. Sie sind unter verschiedenen Winkeln zueinander und zur Längsachse des Nagelschaftes 1 ausgerichtet. Die Längsachse des Nagelschaftes ist in der Zeichnung nicht dargestellt. Sie verläuft in Längsrichtung des Nagelschaftes und bildet seine Symmetrieachse. Um diese Längsachse ist der Nagelschaft abgesehen von den Bohrungen 5 und 7 und sonstiger Öffnungen und Ausnehmungen rotationssymmetrisch.

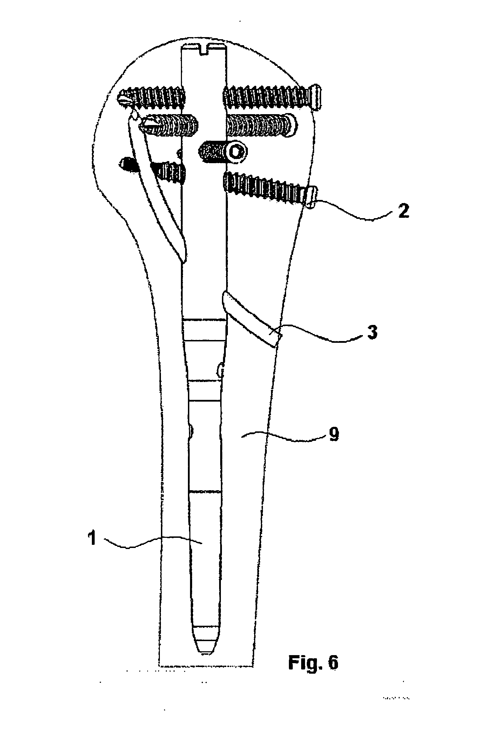

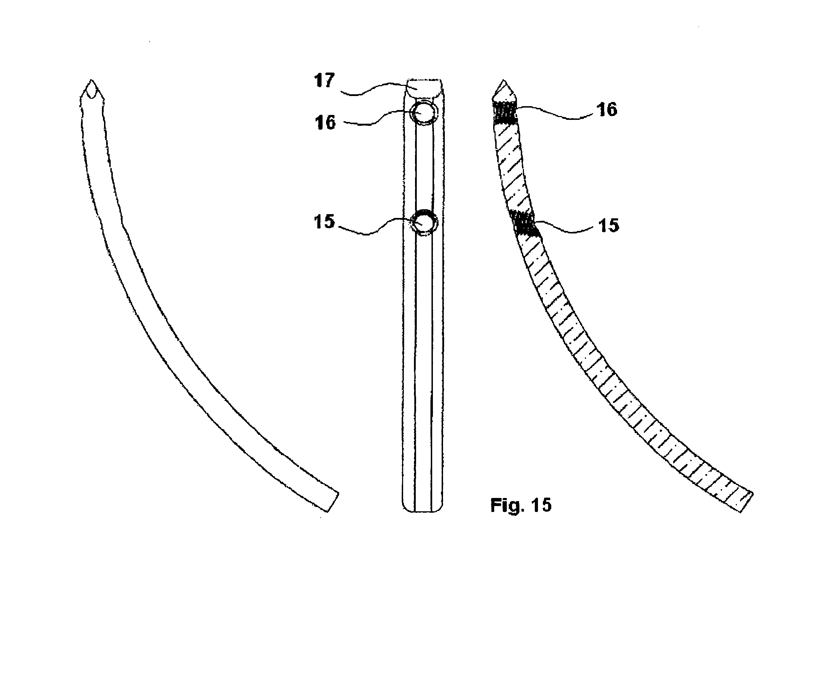

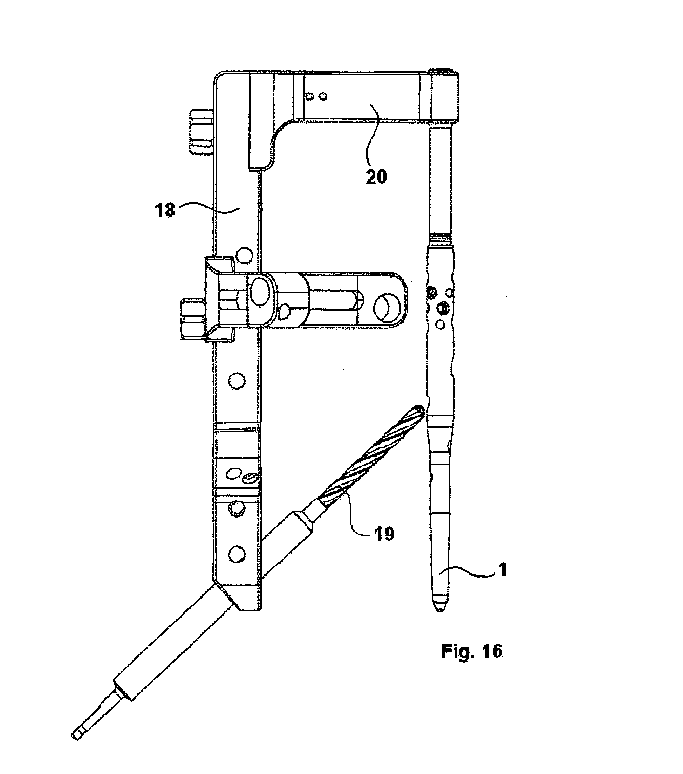

An dem proximalen Ende ist der Nagelschaft mit einer Aufnahme 8 für ein Zielgerät ausgestattet. Ein Zielgerät ist in den Figuren 15 und 16 dargestellt. Mit Hilfe dieses Zielgeräts wird der Humerusnagel in den Humerus eines Patienten eingebracht. Figur 6 zeigt den Humerusnagel zusammen mit einem Abschnitt eines Humerus 9. In dieser Darstellung ist die Anordnung des Humerusnagels im Humerus erkennbar.

Zusätzlich zu den Bohrungen 5 und 7 und der Aufnahme 8 befindet sich eine Durchgangsöffnung 10 in dem Nagelschaft, in welche das Stabilisierungselement 3 eingesetzt ist. Die Position der Durchgangsöffnung ist zwischen den proximalen Bohrungen 5 und den distalen Bohrungen 7. Die Durchgangsöffnung 10 erstreckt sich durch den Nagelschaft 1 hindurch und schneidet die Längsachse. Sie weist über ihre gesamte Länge denselben Querschnitt auf. Der Verlauf der Durchgangsöffnung 10 wird durch eine Kurve repräsentiert. Diese Kurve ist identisch mit der Kurve 22, die den Verlauf des Stabilisierungselements 3 repräsentiert. Die Kurve verläuft durch das Zentrum bzw. die Mitte der Durchgangsöffnung und erstreckt sich in Längsrichtung vom Eingang auf der einen Seite des Nagelschaftes 1 bis zum Ausgang der Durchgangsöffnung an der anderen Seite des Nagelschaftes 1. Der Verlauf der Kurve 22 ist in den Figuren 3 und 4 dargestellt. Der Winkel zwischen der Längsachse des Nagelschaftes 1 einerseits und einer Tangente an die Kurve im Schnittpunkt zwischen der Kurve und der Längsachse andererseits beträgt 36°.

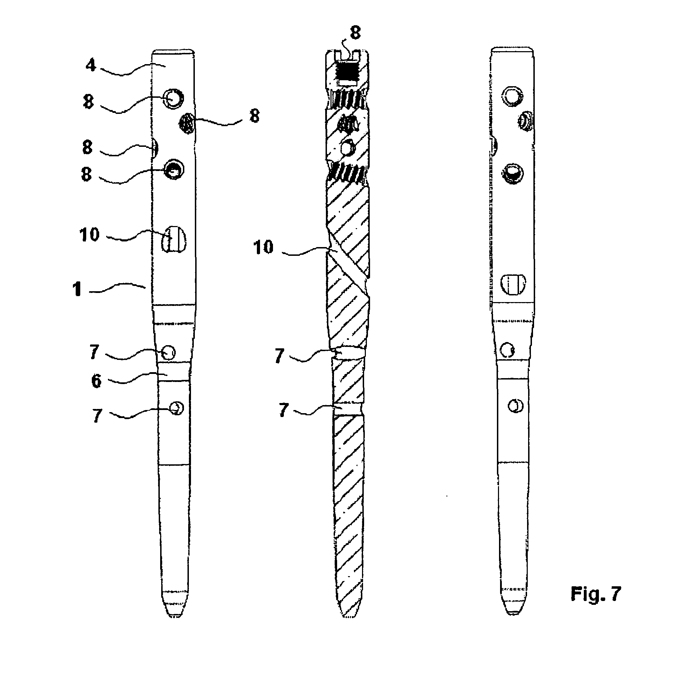

Der Nagelschaft 1 ist in Figur 7 in verschiedenen Ansichten dargestellt. Die rechte und linke Darstellung zeigen eine Seitenansicht, wobei die Aufsicht auf die Seite in der linken Darstellung in die entgegengesetzte Richtung wie bei der rechten Darstellung. Der Nagelschaft 1 ist in der rechten Darstellung gegenüber der linken Darstellung um 180° gedreht. Die mittlere Darstellung zeigt einen Längsschnitt durch den Nagelschaft wobei die Längsachse in der Schnittebene liegt. In diesen Darstellungen sind besonders gut erkennbar der proximale Bereich 4 mit den proximalen Bohrungen 5, der distale Bereich 6 mit den distalen Bohrungen 7, die Aufnahme 8 für ein Werkzeug an der proximalen Stirnseite und die Durchgangsöffnung 10 für die Aufnahme eines in Figur 7 nicht dargestellten Stabilisierungselements.

Das Stabilisierungselement 3 folgt dem Verlauf der Durchgangsöffnung 10 und weist dieselbe Krümmung auf, die durch die Kurve 22 in den Figuren 3 und 4 repräsentiert wird. An einem Ende ist in dem Stabilisierungselement 3 als Aufnahme 11 eine Gewindebohrung vorgesehen. Es handelt sich hierbei um eine Gewindebohrung, welche das Stabilisierungselement vollständig durchdringt. Sie dient der Aufnahme eines der als Knochenschrauben ausgebildeten Fixierungselemente 2. Das dem Schraubenkopf abgewandte Ende des Fixierungselementes 2 ist in der Gewindebohrung angeordnet. Der sich an die Aufnahme 11 anschließende Endbereich weist eine keilförmige Schneide 12 auf. Sie erleichtert das Einsetzen des Stabilisierungselements in den Knochen eines Patienten und das Einführen des Stabilisierungselements 3 in die Durchgangsöffnung 10 des Nagelschaftes 1.

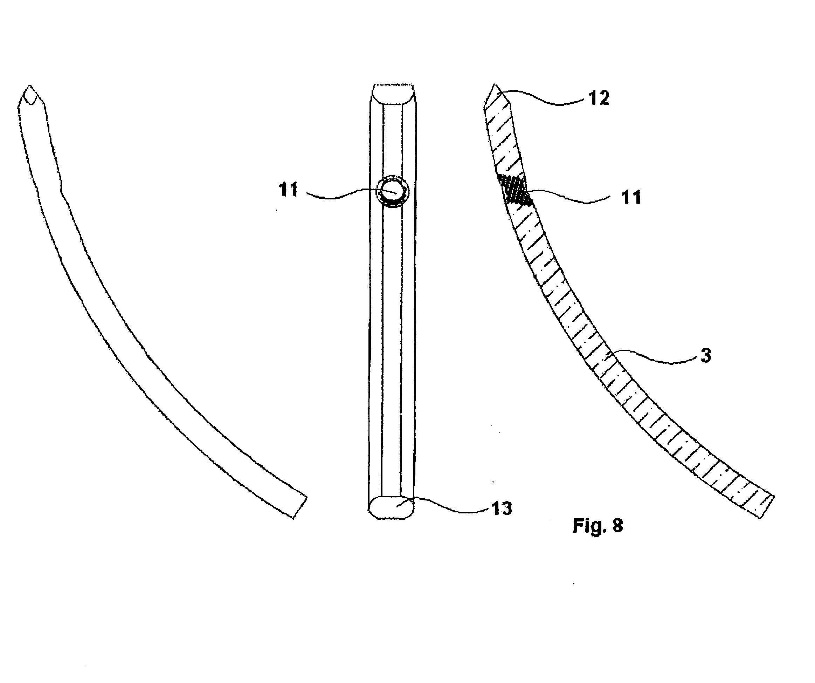

In Figur 8 ist das Stabilisierungselement in verschiedenen Ansichten dargestellt. Die rechte Ansicht zeigt den länglichen Querschnitt 13 des Stabilisierungselements 3.

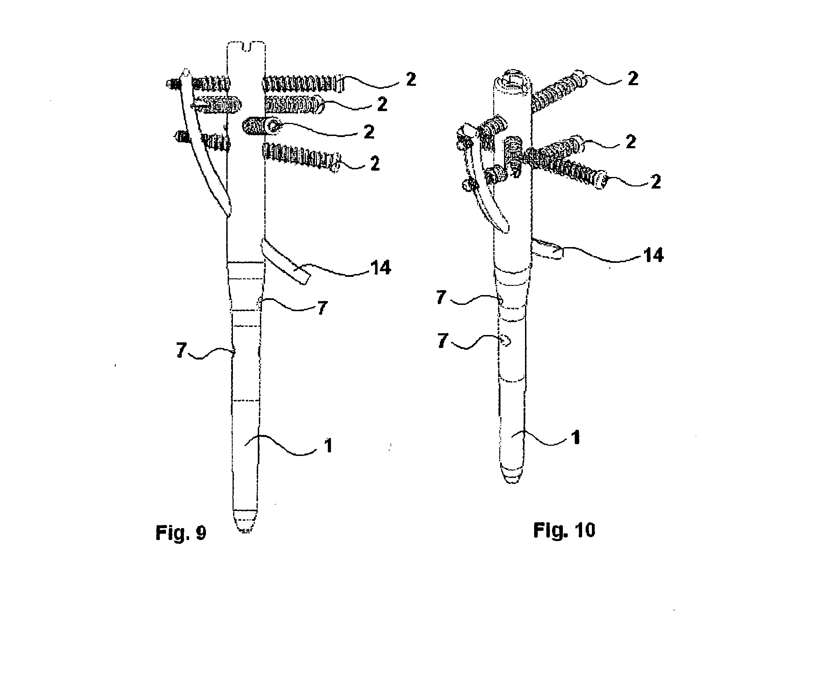

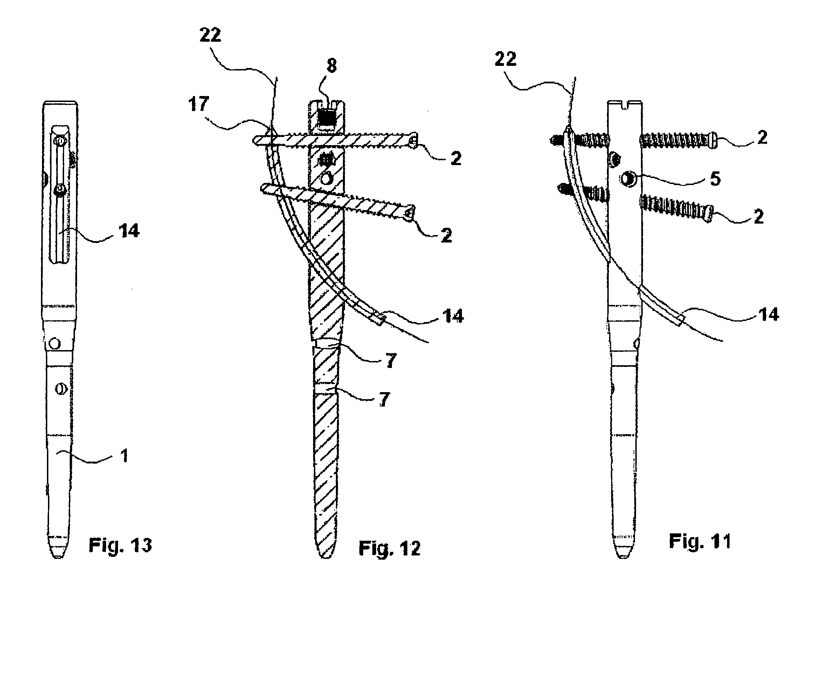

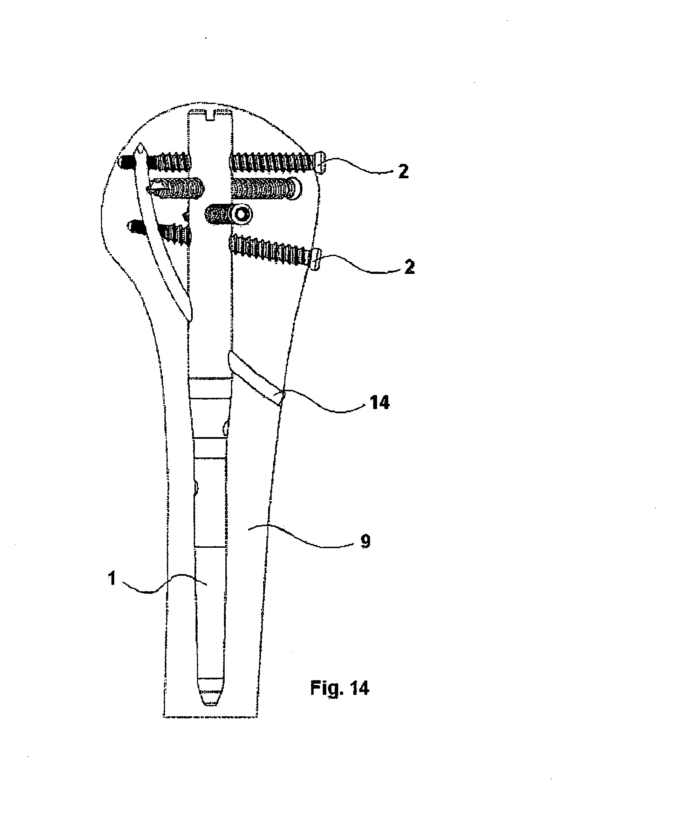

In den Figuren 9 bis 15 ist ein zweites Ausführungsbeispiel eines Humerusnagels dargestellt. Der Nagelschaft 1 und die Fixierungselemente 2 sind identisch mit dem ersten Ausführungsbeispiel. Sie sind daher mit denselben Bezugszahlen versehen. Das zweite Ausführungsbeispiel unterscheidet sich hinsichtlich des Stabilisierungselements 14 vom ersten Ausführungsbeispiel. Das Stabilisierungselement 14 ist länger als das Stabilisierungselement 3 des ersten Ausführungsbeispiels. Ferner weist das Stabilisierungselement 14 zwei als Gewindebohrungen ausgestaltete Aufnahmen 15 und 16 für Fixierungselemente 2 auf. Eine erste Aufnahme 15 ist für dasselbe Fixierungselement 2 wie die Aufnahme 11 des Stabilisierungselements 3 des ersten Ausführungsbeispiels. Die zweite Aufnahme 16 ist untermittelbar im Anschluss an die keilförmige Schneide 17 des Stabilisierungselements 14 vorgesehen. Beide Aufnahmen 15 und 16 durchdringen das Stabilisierungselement. Durch das längere Stabilisierungselement 14 wird eine noch höhere Stabilität der Versorgung der Humerusfraktur erreicht. Der Verlauf des Stabilisierungselements 14 wird durch dieselbe Kurve 22 repräsentiert wie der Verlauf des Stabilisierungselements 3 gemäß erstem Ausführungsbeispiel.

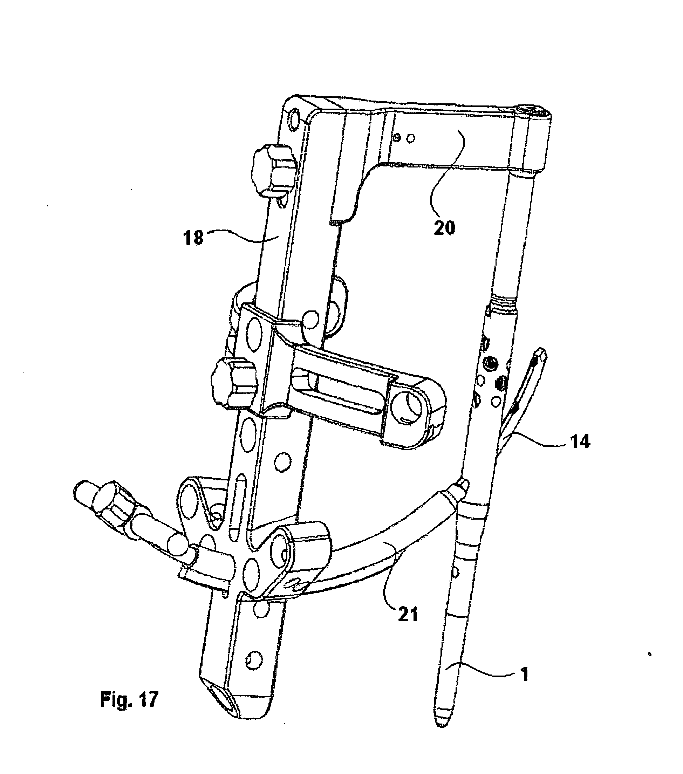

Die Figuren 16 und 17 zeigen ein Zielgerät 18 zum Einbringen eines Humerusnagels in den Humerus eines Patienten. In Figur 16 ist das Zielgerät 18 in Seitenansicht mit einem Bohrer 19 dargestellt. Der Bohrer 19 dient dazu, ein Loch für ein Stabilisierungselement 3 oder 14 in den Humerus zu bohren. Dabei wird der Nagelschaft 1 eines Humerusnagels an einem Zielarm 20 des Zielgeräts 18 über die Aufnahme 8 befestigt. Die Aufnahme 8 eines Nagelschaftes ist in den Figuren 2, 4, 9 und 11 erkennbar. Figur 17 zeigt das Zielgerät 18 in perspektivischer Ansicht mit einer Führungshülse 21 für ein Stabilisierungselement 14. Die Führungshülse 21 sorgt dafür, dass das Stabilisierungelement 14 unter der durch die Durchgangsöffnung 10 des Nagelschaftes vorgesehenen Krümmung in den Humerus eingeführt wird. Das Einbringen des Stabilisierungselementes 14 wird dadurch erleichtert.

### Bezugszahlen

- 1: Nagelschaft
- 2: Fixierungselement
- 3: Stabilisierungselement
- 4: proximaler Bereich des Nagelschaftes
- 5: proximale Bohrung
- 6: distaler Bereich des Nagelschaftes
- 7: distale Bohrung
- 8: Aufnahme für ein Werkzeug
- 9: Humerus
- 10: Durchgangsöffnung
- 11: Aufnahme
- 12: keilförmige Schneide
- 13: länglicher Querschnitt des Stabilisierungselements
- 14: Stabilisierungselement
- 15: Aufnahme
- 16: Aufnahme
- 17: keilförmige Schneide
- 18: Zielgerät
- 19: Bohrer
- 20: Zielarm
- 21: Führungshülse
- 22: Verlauf des Stabilisierungselements des ersten Ausführungsbeispiels

## Patentansprüche

1. Humerusnagel zur Versorgung von Frakturen des Humerus mit einem länglichen Nagelschaft (1),
mit mehreren Bohrungen (5) im proximalen Bereich (4) zur Aufnahme von stiftförmigen Fixierungselementen (2),
mit einer Durchgangsöffnung (10) in dem Nagelschaft (1) zur Aufnahme eines Stabilisierungselements (3, 14),
mit einem Stabilisierungselement (3, 14) zum Einsetzen in die Durchgangsöffnung (10),
mit mindestens einer Aufnahme (11) in dem Stabilisierungselement (3, 14) für einen Abschnitt eines in einer der Bohrungen (5) angeordneten Fixierungselements (2), um das Stabilisierungselement (3, 14) mit einem Fixierungselement (2) zu verbinden,
**dadurch gekennzeichnet,**
**dass** das Stabilisierungselernent (3, 14) einen gekrümmten Verlauf (22) aufweist.

2. Humerusnagel nach Anspruch 1, **dadurch gekennzeichnet, dass** die Durchgangsöffnung (10) zwischen den Bohrungen (5) im proximalen Bereich (4) und dem distalen Ende durch den Nagelschaft (1) verläuft.

3. Humerusnagel nach Anspruch 1 oder 2, **dadurch gekennzeichnet, dass** die Durchgangsöffnung (10) einen gekrümmten Verlauf (22) aufweist.

4. Humerusnagel nach einem der vorhergehenden Ansprüche, **dadurch gekennzeichnet, dass** die Durchgangsöffnung (10) über ihre gesamte Länge einen konstanten Querschnitt aufweist.

5. Humerusnagel nach Anspruch 4, **dadurch gekennzeichnet, dass** der Querschnitt der Durchgangsöffnung (10) länglich ist.

6. Humerusnagel nach einem der vorhergehenden Ansprüche, **dadurch gekennzeichnet, dass** die Längsachse mindestens einer der Bohrungen (5) und eine durch den Verlauf der Durchgangsöffnung (10) vorgegebene Kurve (22) einen Schnittpunkt aufweisen.

7. Humerusnagel nach einem der vorhergehenden Ansprüche, **dadurch gekennzeichnet, dass** das Stabilisierungselement (3, 14) länglich ist.

8. Humerusnagel nach Anspruch 3, **dadurch gekennzeichnet, dass** der Verlauf (22) des Stabilisierungselements (3, 14) qualitativ mit dem gekrümmten Verlauf der Durchgangsöffnung (10) übereinstimmt.

9. Humerusnagel nach einem der vorhergehenden Ansprüche, **dadurch gekennzeichnet, dass** die Aufnahme (11) des Stabilisierungselements (3, 14) als Bohrung ausgestaltet ist.

10. Humerusnagel nach einem der vorhergehenden Ansprüche, **dadurch gekennzeichnet, dass** sich die Aufnahme (11) an einem Endbereich des Stabilisierungselements (3, 14) befindet.

11. Humerusnagel nach einem der vorhergehenden Ansprüche, **dadurch gekennzeichnet, dass** das Stabilisierungselement (3, 14) an einem Endbereich einen Abschnitt aufweist, dessen Querschnitt größer ist als der Querschnitt der Durchgangsöffnung.

12. Humerusnagel nach einem der vorhergehenden Ansprüche, **dadurch gekennzeichnet, dass** das Stabilisierungselement (3, 14) einen länglichen Querschnitt aufweist.

13. Humerusnagel nach einem der vorhergehenden Ansprüche 12, **dadurch gekennzeichnet, dass** das Stabilisierungselement (3, 14) an einem Ende eine keilförmige Schneide (12) aufweist.

## Claims

1. Humeral nail for caring for fractures of the humerus with an oblong nail shank (1),
with several holes (5) in the proximal area (4) for holding pin-shaped fixing elements (2),
with a through opening (10) in the nail shank (1) for holding a stabilising element (3, 14),
with a stabilising element (3, 14) for insertion in the through opening (10), with at least one support (11) in the stabilising element (3, 14) for a section of a fixing element (2) arranged in one of the holes (5) to connect the stabilising element (3, 14) with a fixing element (2),
**characterised in that**
the stabilising element (3, 14) has a curved course (22).

2. Humeral nail according to claim 1, **characterised in that** the through opening (10) runs through the nail shank between the holes (5) in the proximal area (4) and the distal end.

3. Humeral nail according to claim 1 or 2, **characterised in that** the through opening (10) has a curved course (22).

4. Humeral nail according to one of the previous claims, **characterised in that** the through opening (10) has a constant cross-section along its entire length.

5. Humeral nail according to claim 4, **characterised in that** the cross-section of the through opening (10) is oblong.

6. Humeral nail according to one of the previous claims, **characterised in that** the longitudinal axis of at least one of the holes (5) and a curve (22) specified by the course of the through opening (10) have a point of intersection.

7. Humeral nail according to one of the previous claims, **characterised in that** the stabilising element (3, 14) is oblong.

8. Humeral nail according to claim 3, **characterised in that** the course (22) of the stabilising element (3, 14) is consistent with the curved course of the through opening (10) in terms of quality.

9. Humeral nail according to one of the previous claims, **characterised in that** the support (11) of the stabilising element (3, 14) is designed as a hole.

10. Humeral nail according to one of the previous claims, **characterised in that** the support (11) is located in the area of an end of the stabilising element (3, 14).

11. Humeral nail according to one of the previous claims, **characterised in that** the stabilising element (3, 14) has a section in the area of an end with a cross-section that is larger than the cross-section of the through opening.

12. Humeral nail according to one of the previous claims, **characterised in that** the stabilising element (3, 14) has an oblong cross-section.

13. Humeral nail according to one of the previous claims 12, **characterised in that** the stabilising element (3, 14) has a wedge-shaped edge (12) on one end.

## Revendications

1. Clou huméral destiné au traitement de fractures de l'humérus avec une tige de forme allongée (1),
avec plusieurs alésages (5) au niveau de l'extrémité proximale (4) destinés à recevoir des éléments de fixation en forme de cheville (2),
avec un trou de passage (10) dans la tige (1) destiné à recevoir un élément de stabilisation (3, 14),
avec un élément de stabilisation (3, 14) destiné à être mis en place dans le trou de passage (10),
avec au moins un logement (11) dans l'élément de stabilisation (3, 14), destiné à recevoir une partie d'un élément de fixation (2) disposé dans un des alésages (5) afin de relier l'élément de stabilisation (3, 14) à un élément de fixation (2),
**caractérisé en ce que**
l'élément de stabilisation (3, 14) présente une forme arquée (22).

2. Clou huméral selon la revendication 1, **caractérisé en ce que** le trou de passage (10) traverse la tige (1) entre les alésages (5) au niveau de l'extrémité proximale (4) et l'extrémité distale.

3. Clou huméral selon la revendication 1 ou 2, **caractérisé en ce que** le trou de passage (10) présente une forme arquée (22).

4. Clou huméral selon l'une quelconque des revendications précédentes, **caractérisé en ce que** le trou de passage (10) présente une section constante sur toute sa longueur.

5. Clou huméral selon la revendication 4, **caractérisé en ce que** la section du trou de passage (10) est de forme allongée.

6. Clou huméral selon l'une quelconque des revendications précédentes, **caractérisé en ce que** l'axe longitudinal d'au moins un des alésages (5) et une courbe (22) définie par la forme du trou de passage (10) présentent un point d'intersection.

7. Clou huméral selon l'une quelconque des revendications précédentes, **caractérisé en ce que** l'élément de stabilisation (3, 14) est de forme allongée.

8. Clou huméral selon la revendication 3, **caractérisé en ce que** la forme (22) de l'élément de stabilisation (3, 14) correspond à la forme arquée du trou de passage (10).

9. Clou huméral selon l'une quelconque des revendications précédentes, **caractérisé en ce que** le logement (11) de l'élément de stabilisation (3, 14) est un alésage.

10. Clou huméral selon l'une quelconque des revendications précédentes, **caractérisé en ce que** le logement (11) se situe au niveau d'une extrémité de l'élément de stabilisation (3, 14).

11. Clou huméral selon l'une quelconque des revendications précédentes, **caractérisé en ce que** l'élément de stabilisation (3, 14) présente, au niveau d'une extrémité, une partie dont la section est plus grande que la section du trou de passage.

12. Clou huméral selon l'une quelconque des revendications précédentes, **caractérisé en ce que** l'élément de stabilisation (3, 14) présente une section de forme allongée.

13. Clou huméral selon l'une quelconque des revendications précédentes 12, **caractérisé en ce que** l'élément de stabilisation (3, 14) présente, au niveau d'une extrémité, une arête cunéiforme (12).
